# FASCICULE DE BREVET EUROPEEN

(11) **EP 3 212 801 B1**
(45) Date de publication et mention de la délivrance du brevet: **29.04.2020**
(21) Numéro de dépôt: 15791710.5
(22) Date de dépôt: 22.10.2015
(51) Int. Cl.: C12Q 1/04, G01N 33/68, G01N 33/493, H01J 49/00

(54) **PROCÉDÉ D'IDENTIFICATION D'UN MICROBE DANS UN PRÉLÈVEMENT CLINIQUE**
VERFAHREN ZUR IDENTIFIZIERUNG EINER MIKROBE IN EINER KLINISCHEN PROBE
METHOD FOR IDENTIFYING A MICROBE IN A CLINICAL SAMPLE

(30) Priorité: 27.10.2014 FR 1460311
(43) Date de publication de la demande: 06.09.2017
(73) Titulaire: Fondation Méditerranée Infection, 13005 Marseille (FR); Université d'Aix Marseille, 13284 Marseille Cedex 07 (FR); Assistance Publique Hôpitaux de Marseille, 13005 Marseille (FR); Centre National de la Recherche Scientifique (C.N.R.S.), 75016 Paris (FR); Institut National de la Santé et de la Recherche Médicale (INSERM), 75013 Paris (FR)
(72) Inventeur: FENOLLAR, Florence, F-13008 Marseille (FR); RAOULT, Didier, F-13008 Marseille (FR); LA SCOLA, Bernard, F-13790 Chateauneuf Le Rouge (FR); CHABRIERE, Eric, F-13009 Marseille (FR); FLAUDROPS, Christophe, F-13009 Marseille (FR); PINAULT, Lucile, F-13012 Marseille (FR)
(74) Mandataire: Cabinet Beau de Loménie
(86) Numéro de dépôt international: PCT/FR2015/052840
(87) Numéro de publication internationale: WO 2016/066930

(56) Documents cités:
- WO-A1-2009/065580
- LAU SUSANNA K P ET AL: "Matrix-assisted laser desorption ionisation time-of-flight mass spectrometry for identification of clinically significant bacteria that are difficult to identify in clinical laboratories.", JOURNAL OF CLINICAL PATHOLOGY, vol. 67, no. 4, avril 2014 (2014-04), pages 361-366, XP009185570, ISSN: 1472-4146
- A. E. CLARK ET AL: "Matrix-Assisted Laser Desorption Ionization-Time of Flight Mass Spectrometry: a Fundamental Shift in the Routine Practice of Clinical Microbiology", CLINICAL MICROBIOLOGY REVIEWS, vol. 26, no. 3, juillet 2013 (2013-07), pages 547-603, XP055204625, ISSN: 0893-8512, DOI: 10.1128/CMR.00072-12
- H. L. KOHLING ET AL: "Direct identification of bacteria in urine samples by matrix-assisted laser desorption/ionization time-of-flight mass spectrometry and relevance of defensins as interfering factors", JOURNAL OF MEDICAL MICROBIOLOGY, vol. 61, no. 3, 23 janvier 2012 (2012-01-23), pages 339-344, XP055204445, ISSN: 0022-2615, DOI: 10.1099/jmm.0.032284-0 cité dans la demande
- M. L. DEMARCO ET AL: "Diafiltration MALDI-TOF Mass Spectrometry Method for Culture-Independent Detection and Identification of Pathogens Directly From Urine Specimens", AMERICAN JOURNAL OF CLINICAL PATHOLOGY, vol. 141, no. 2, 16 janvier 2014 (2014-01-16), pages 204-212, XP055204398, ISSN: 0002-9173, DOI: 10.1309/AJCPQYW3B6JLKILC cité dans la demande
- SASCHA SAUER ET AL: "Classification and Identification of Bacteria by Mass Spectrometry and Computational Analysis", PLOS ONE, vol. 3, no. 7, 30 juillet 2008 (2008-07-30), page e2843, XP055114067, DOI: 10.1371/journal.pone.0002843

## Description

La présente invention concerne une méthode d'identification d'un microbe dans un prélèvement clinique par comparaison de spectres de masse réalisés par spectrométrie de masse de type MALDI-TOF et une banque de spectres de référence d'une pluralité d'extraits protéiques d'échantillons contenant chacun un microbe parmi respectivement une pluralité de microbes de référence. Plus particulièrement, on y décrit la réalisation d'une banque de données spécifiques de spectres de masse de type MALDI-TOF réalisés sur des extraits de prélèvements humains, tels que les urines infectées par des microbes, afin de pouvoir réaliser directement le diagnostic spécifique de l'infection sans étape d'isolement du microbe.

Actuellement, en routine, la détection et l'identification de microorganismes à partir de prélèvements de patients sont réalisées en 2 étapes:
- une étape initiale d'isolement des microorganismes après ensemencement du prélèvement du patient en milieu de culture solide (gélose) ou liquide et une incubation minimale 24 heures. Ce délai peut être allongé lorsque la culture est fastidieuse. L'objectif étant d'obtenir des colonies isolées des microorganismes présents dans les prélèvements de patients sur milieu de culture solide.
- une étape d'identification des colonies isolées en quelques minutes par spectrométrie de masse de type MALDI-TOF à partir de banques de données comportant les spectres des microorganismes obtenus à partir de colonies isolées sur milieu de culture solide.

La détection des microorganismes à partir des prélèvements des patients peut être réalisée par l'examen au microscope de lames où les prélèvements ont été étalés et colorés par la coloration de Gram. Cette approche manque à la fois de sensibilité et de spécificité. Ce n'est qu'une technique de débrouillage permettant de différencier les bactéries à Gram positif de celles à Gram négatif, mais ne permettant pas de réaliser une identification précise. De plus, la coloration de Gram est opérateur dépendant avec une grande variabilité des résultats observés selon la personne réalisant cet examen.

La détection de microorganismes à partir des prélèvements des patients peut aussi être réalisée par biologie moléculaire. L'utilisation de la biologie moléculaire permet une identification bactérienne rapide et précise dans bien des cas par amplification de l'ADN par PCR. Les avantages de la biologie moléculaire sont nombreux. Cependant, les techniques de biologie moléculaire ne sont pas disponibles dans la plupart des laboratoires de biologie et sont de plus très onéreuses. Elles nécessitent de connaître à l'avance le microorganisme recherché si l'on veut pouvoir l'identifier par PCR spécifique en temps réel en 2-3 heures à partir du prélèvement de patient. Sinon, il faut avoir recours à la PCR couplée au séquençage avec des délais de rendu de résultats beaucoup plus long. De plus, les informations obtenues par le séquençage ne sont pas toujours suffisamment discriminantes pour permettre l'identification au niveau de l'espèce et l'étude d'autres séquences ou gènes cibles est requise, ce qui augmente les délais de réponse. Cette méthodologie nécessite une grande expertise et doit être réservée aux identifications difficiles ou aux microorganismes dont la culture est fastidieuse.

De façon connue, la spectrométrie de masse est une technique physique d'analyse permettant de détecter et d'identifier des molécules d'intérêt par mesure de leur masse. Son principe réside dans la séparation en phase gazeuse de molécules chargées (ions) en fonction de leur rapport masse/charge (m/z). Les applications de la spectrométrie de masse sont très vastes et concernent principalement l'identification de peptides ou de protéines, l'analyse de leur séquence en acides aminés ou encore la mise en évidence de modifications post-traductionnelles.

La spectrométrie de masse sous sa variante MALDI-TOF (matrix-assisted laser desorption ionization-time-of-flight) permet d'identifier et de classifier différents types bactériens en utilisant des bactéries intactes. En effet, parmi les divers composants identifiés dans un spectre de spectrométrie de masse MALDI-TOF, certains semblent spécifiques d'une espèce bactérienne précise. Ainsi, il est possible d'identifier un grand nombre d'espèces bactériennes à partir de colonies isolées en culture de prélèvements de patients en utilisant des banques de données fabriquées grâce à l'analyse des colonies de chacune de ces espèces. La spectrométrie de masse de type MALDI-TOF s'est imposée ces dernières années dans les laboratoires de microbiologie clinique pour l'identification des colonies de bactéries ou de levures isolées sur milieu de culture solide à partir de prélèvements humains. En effet, ses atouts sont nombreux : (1) rapidité de l'identification (en quelques minutes) ; (2) précision de l'identification (sans nécessité préalable de connaître le type de microorganisme recherché) ; et (3) faible coût par échantillon (moins de 50 centimes d'euros par échantillon).

Parmi les fabricants de spectromètre de masse, deux systèmes MALDI-TOF complets sont disponibles à la vente, commercialisés par les sociétés Bruker Daltonics (Allemagne) et Biomérieux (France). Chaque instrument est accompagné d'un logiciel de pilotage, d'une banque de données obtenue à partir de colonies isolées en milieu solide et d'un système expert permettant l'identification du microorganisme reposant sur l'analyse du spectre généré par le spectromètre de masse. Une application de cette technologie à l'identification des bactéries implique actuellement l'isolement de colonies (obtenues après 24h - 48h de culture) et l'analyse de bactéries entières ou d'extraits protéiques de bactéries isolées, les dites bactéries étant isolées directement à partir des prélèvements de patients.

Le gain de temps est important pour la mise en route d'une antibiothérapie.

Le but de la présente invention est de fournir une méthode d'identification des microorganismes directement à partir de prélèvements de patients, sans étape de culture, afin de pouvoir obtenir dans des délais très rapides l'identification de microorganismes pathogènes dans les prélèvements de patients. Ceci permettrait de pouvoir adapter le traitement antibiotique le plus rapidement possible afin de réduire la morbidité, mortalité et le coût de la prise en charge du patient.

Plus particulièrement, un but de la présente invention est de tester des prélèvements d'urines de patients présentant des infections urinaires. En effet, les infections urinaires sont les infections bactériennes les plus fréquentes quel que soit l'âge. Les prélèvements urinaires correspondent à plus de 40% des prélèvements reçus au laboratoire de microbiologie clinique d'un hôpital (la proportion de prélèvements urinaires reçus dans un laboratoire de ville étant encore plus élevée). Les principales bactéries retrouvées comme responsable d'infections urinaires sont : *Escherichia coli (48%), Klebsiella pneumoniae (10%), Enterococcus faecalis (8%), Proteus mirabilis (5%)* et *Pseudomonas aeruginosa (5%).*

Quelques études discutées ci-après ont été réalisées sur l'identification de microorganismes directement à partir des prélèvements de patients comprenant des urines principalement, sans culture pré-analytique, par spectrométrie de masse MALDI-TOF par comparaison avec des spectres de référence de la base de données Biotyper réalisé à partir d'isolats bactériens cultivés, voir par exemple WO 2009/065580 A1. Mais, les résultats obtenus ne sont pas concluants voire pas satisfaisants.

Dans Ferreira et al (Clinical Microbiology and infection, vol. 17, number 7, july 2011, pages 1007-1012) l'étude portait quasi-exclusivement sur des échantillons d'urines avec des infections urinaires à *Escherichia coli* (75% cas étudiés) avec en outre une présélection de patients avec une urine ayant une concentration supérieure à >10⁵ CFU/mL voir >5x10⁵ CFU/mL pour certains pathogènes tels que *E. faecalis* et un volume de 4 ml était nécessaire à la procédure, cependant ce volume est parfois difficile à obtenir notamment chez les nourrissons.

Dans Rossello et al (APMIS 122 (2013) pages 790-795), seulement 80 prélèvements d'urines de patients dont 60 avec *Escherichia coli* (75% cas étudiés) et nécessitant 5 mL urine (pas toujours disponibles). Enfin, il est recommandé une préincubation du culot bactérien dans un milieu de culture pendant 4 heures pour les échantillons <10⁷ CFU/mL ce qui correspond à la majorité des patients.

Dans DeMarco et al (Am. J. Clin. Pathol. 2014 141:204-212), l'étude repose sur l'analyse d'un échantillon d'urines d'un volume minimum de 15 mL auxquels des microorganismes *(Escherichia coli* et *Staphylococcus saprophyticus,* notamment) ont été artificiellement ajoutées. L'étude réelle (à partir d'échantillon de patient infecté) a été réalisée seulement sur 15 patients présentant des infections à *Escherichia coli, Klebsiella pneumoniae* et *Proteus mirabilis* avec une limite de détection de > 10⁵ CFU/mL.

Dans Burillo et al (PLoS One 2014 9 :e86915), l'étude repose sur l'analyse d'un échantillon d'urines minimum de 15 mL et elle était limitée aux échantillons contenant >10⁵ CFU/mL. De plus, l'interprétation ne respectait pas toujours un score de validation des spectres > 1.99, ce qui ne correspond pas aux critères exigés par le fabricant.

Dans Sanchez-Juanes et al (JJ. Clin. Microbiol. 2014, 52(1):335), l'étude porte sur des prélèvements de 3 mL d'urine. Enfin, elle est aussi limitée à des prélèvements de seulement 71 patients contenant > 10⁵ CFU/mL.

Cependant, en pratique pour être fiable, l'identification doit pouvoir porter sur des extraits protéiques obtenus à partir de prélèvements d'urines de pas plus de 1ml et avec des concentrations présentes dans les échantillons des prélèvements cliniques infectés chez les patients humains, à partir de seulement 10⁴ bactéries/mL pour les urines.

En outre, dans tous ces articles, l'acide formique, qui est un produit dangereux, a été utilisé comme moyen de lyse chimique dans le traitement de préparation d'un extrait protéique.

Les résultats d'identification des microorganismes par spectrométrie de masse de type MALDI-TOF sur des échantillons d'extraits protéiques obtenus à partir de prélèvements urinaires de patients, à l'aide des logiciels et banques de spectres de références disponibles, notamment le logiciel et la base de données BIOTYPER® de la société BRUKER DALTONICS® ne comprenant que des spectres de référence de colonies isolées, rapportés ci-après sur des prélèvements de 1ml, sont médiocres avec moins de 50% d'identification.

On a découvert selon la présente invention qu'il est possible d'identifier de microorganismes directement à partir d'extraits protéiques de prélèvements de patients, sans culture ou autre phase pré-analytique, par spectrométrie de masse MALDI-TOF, à partir de prélèvements d'urines de pas plus de 1ml et avec des concentrations présentes dans les échantillons des prélèvements cliniques infectés chez les patients humains, à partir de à 10⁴/ml pour les urines, sous réserve de réaliser et comparer les spectres de l'échantillon à analyser et des spectres de référence dans certaines conditions sans étape d'isolement et culture du microbe, à savoir à l'aide de spectres de référence réalisés à partir de prélèvements cliniques infectés sans isolement et/ou culture des microorganismes comme explicité ci-après.

Du fait que le contenu protéique des bactéries est modifié par l'environnement et interaction avec les autres composants chimiques ou biologiques dudit prélèvement clinique, le spectre d'un extrait protéique dans un prélèvement clinique du microorganisme contenant un microbe donné diffère du spectre réalisé sur un extrait protéique obtenu à partir d'une colonie cultivée purifiée d'un échantillon microbien purifié. Il en résulte que l'identification selon la présente invention à l'aide de spectres de référence réalisés à partir de prélèvements cliniques infectés entiers sans isolement et/ou culture des microorganismes qu'il contient, sont plus fiables et permettent un meilleur taux d'identification en comparaison avec une banque de spectres réalisés sur des microorganismes purifiés et sont aussi performants que les identifications réalisées à partir de spectres d'échantillons d'extraits protéiques bactériens obtenus et analysés après isolement et/ou culture desdits bactéries qu'ils contiennent en comparaison avec des spectres obtenus à partir d'échantillons de bactéries purifiées.

La présente invention permet une identification beaucoup plus rapide en moins d'une heure car ne nécessitant pas la perte de temps de 24 à 48h pour l'isolement et/ou la culture du microorganisme contenu dans le prélèvement clinique ce qui en cas d'infection peut s'avérer un gain de temps décisif dans le traitement thérapeutique.

Plus précisément, la présente invention fournit un procédé d'identification d'un microbe dans un prélèvement clinique par comparaison de spectres de masse réalisés par spectrométrie de masse de type MALDI-TOF d'un échantillon d'extrait protéique du dit prélèvement à analyser contenant un dit microbe, par comparaison avec des spectres de référence d'au moins une banque de spectres d'une pluralité d'extraits protéiques d'échantillons contenant chacun un microbe choisi parmi respectivement une pluralité de microbes de référence, caractérisé en ce qu'on réalise les étapes dans lesquelles :
a) on établit une banque de spectres de référence par spectrométrie de masse de type MALDI-TOF, lesdits spectres de référence étant réalisés sur des échantillons de référence d'extraits protéiques obtenus selon un même procédé de préparation à partir de prélèvements cliniques de référence de même nature comprenant un traitement de lyse desdits microbes, sans isolement et/ou sans culture desdits microbes desdits prélèvements cliniques de référence, et
b) on réalise un spectre d'un échantillon à analyser d'extrait protéique obtenu à partir d'un prélèvement clinique à analyser de même nature que celui des échantillons de référence, ledit échantillon à analyser étant obtenu selon le même dit procédé de préparation à partir dudit prélèvement clinique comprenant un traitement de lyse des microbes éventuels qu'il est susceptible de contenir, sans isolement et/ou sans culture dudit microbe dudit prélèvement clinique à analyser, et
c) on détermine que ledit prélèvement clinique à partir duquel un échantillon à analyser a été obtenu, contient un microbe de référence donné choisi parmi ladite pluralité des différents microbes de référence de ladite banque de référence si on retrouve dans le spectre de l'échantillon à analyser de l'étape b) l'ensemble des pics caractéristiques d'un dit spectre de référence d'un échantillon de référence contenant ledit microbe de référence donné.

Plus particulièrement, ladite nature desdits prélèvements cliniques consiste dans des urine, liquide céphalo-rachidien, lavage broncho-alvéolaire ou sang.

De préférence, à l'étape a), pour chaque microbe de référence, la banque comprend une pluralité de spectres réalisés sur une pluralité d'échantillons obtenus à partir de respectivement une pluralité de catégories distinctes de prélèvements cliniques d'une même nature de prélèvement clinique, les différentes catégories distinctes de prélèvements cliniques d'une même nature pour un même microbe de référence correspondant de préférence à des prélèvements cliniques dont les composants autres que le microbe au sein dudit prélèvement clinique sont qualitativement et/ou quantitativement différents induisant des différences spectrales de tout ou partie de pics caractéristiques des protéines ou fragments de protéines provenant dudit microbe de référence.

Plus particulièrement, les dits spectres de référence sont obtenus à partir de différentes catégories distinctes de prélèvements d'urines comprenant des concentrations en globules blancs et/ou globules rouges différentes, avec de préférence au moins :
- un premier groupe de prélèvements d'urines contenant pas plus de 10 globules blancs et/ou globules rouges /µl, notamment correspondant aux patients avec une infection urinaire et une faible réponse inflammatoire locale, et
- un deuxième groupe de prélèvements d'urines contenant plus de 10 globules blancs et/ou globules rouge /µl, notamment correspondant aux patients avec une infection urinaire et une réponse inflammatoire locale élevée.

Plus particulièrement, lesdits microbes de référence sont choisis parmi les bactéries et les levures, de préférence les bactéries.

Plus particulièrement encore, lesdites bactéries de référence comprennent au moins *Escherichia coli*, *Klebsiella pneumoniae, Enterococcus faecalis, Proteus mirabilis, Pseudomonas aeruginosa.*

Une banque de données de spectres de référence de prélèvements d'urines doit permettre de pouvoir détecter et identifier directement dans les prélèvements des patients les microorganismes pathogènes responsables d'infections urinaires.

Plus particulièrement encore, lesdites bactéries de référence, que l'on retrouve notamment dans les infections urinaires, sont choisies parmi au moins *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii.*

Plus particulièrement encore, lesdits microbes de référence comprennent au moins : *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii, Pseudomonas putida Serratia marcescens*, *Klebsiella oxytoca, Aerococcus urinae ; Staphylococcus capitis ; Pseudomonas putida, Acinetobacter nosocomialis, Enterobacter kobei, Streptococcus oralis, Enterococcus gallinarum, Stenotrophomonas maltophilia, Raoultella ornithinolytica, Pseudomonas mosselii, Hafnia alvei, Providencia stuartii, Corynebacterium amycolatum, Corynebacterium striatum, Streptococcus anginosus, Acinetobacter baumannii, Staphylococcus hominis ssp hominis, Gardnerella vaginalis, Candida albicans et Candida glabrata.*

Une banque de données de spectres de prélèvements de liquides broncho-alvéolaires doit permettre de pouvoir identifier directement des microorganismes pathogènes responsables de pneumopathies. Pour une banque de spectres de liquides céphalo-rachidiens, elle doit permettre de mettre en évidence directement dans les prélèvements de patients les microorganismes pathogènes responsables méningites.

Les prélèvements de lavages broncho-alvéolaires comprennent principalement les bactéries *Staphylococcus aureus,* des bacilles à Gram négatif non fermentant tels que *Pseudomonas aeruginosa et Acinetobacter baumanii* et des entérobactéries telles que *Escherichia coli, Klebsiella pneumoniae et Proteus mirabilis.* Les prélèvements de liquides céphalo-rachidiens comprennent principalement des bactéries *Neisseria meningitidis, Streptococcus pneumoniae, Escherichia coli* et autres entérobactéries.

Une autre application de ce type de banques de données est la réalisation d'une banque de données de spectres de flacons de bouillons hémocultures inoculés avec du sang de patients infectés.

De préférence, ladite banque de référence comprend en outre au moins un spectre de référence d'extrait protéique de dit prélèvement clinique sain non infecté par un microbe.

Plus particulièrement, la dite banque de référence comprend au moins 500 spectres de référence réalisés à partir d'au moins 500 échantillons de dits prélèvements cliniques de référence contenant chacun un dit microbe de référence, de préférence l'ensemble desdits prélèvements cliniques de référence contenant au moins 10 dits microbes de référence différents, de préférence au moins 20 dits microbes de référence différents.

Plus particulièrement, la base comprend au moins 1000 spectres de référence réalisés à partir d'au moins 1000 échantillons de dits prélèvements cliniques de référence contenant chacun un dit microbe de référence, l'ensemble desdits prélèvements cliniques de référence contenant au moins 40 dits microbes de référence différents.

Plus particulièrement, lesdits extraits protéiques sont obtenus en effectuant un procédé de préparation comprenant les étapes successives suivantes :
1) on élimine le liquide du dit prélèvement clinique à analyser, et on récupère un premier résidu solide, et
2) de préférence, on ajoute une solution de détergent au dit premier résidu solide, et
3) on élimine le liquide à nouveau et on récupère un deuxième résidu solide, et
4) on réalise une lyse des cellules présente dans ledit deuxième résidu solide, de préférence une lyse mécanique, et
5) on solubilise les protéines et fragments de protéines présents dans le dit deuxième résidu solide, avec une solution de solvant, et
6) on élimine les substances non solubilisées.

L'acide formique, usuellement utilisé pour réaliser une lyse chimique et extraire protéines et peptides à partir des prélèvements de patients est à l'origine d'accident de travail (brûlures de la peau et de lésions oculaires graves) du personnel. Ces accidents ont conduits à réévaluer les protocoles techniques de préparation des extraits protéiques à partir des prélèvements cliniques pour la réalisation des spectres pour par spectrométrie de masse de type MALDI-TOF sans avoir à utiliser ce produit dangereux.

De préférence, lesdits extraits protéiques sont obtenus en effectuant le procédé de préparation comprenant les étapes successives suivantes :
1) on élimine le liquide du dit prélèvement clinique à analyser, en réalisant une première centrifugation et éliminant le surnageant de centrifugation, et en récupérant un premier résidu solide qui est le culot de première centrifugation, et
2) on ajoute une solution de détergent au dit premier résidu solide, de préférence une solution de saponine, et
3) on récupère un deuxième résidu solide par une deuxième centrifugation et en éliminant le surnageant et en récupérant un deuxième résidu solide qui est le culot de deuxième centrifugation, et
4) on réalise une lyse mécanique dudit deuxième résidu solide par broyage mécanique permettant d'obtenir des fragments de taille inférieure ou égale à 1 µm de préférence réalisé à l'aide de microbilles de verre, et
5) on solubilise les protéines et fragments de protéines présents dans le dit deuxième résidu solide de lysat cellulaire, de préférence avec une solution d'acide trifluoro-acétique et acétonitrile, et
6) on élimine les substances non solubilisées, par une troisième centrifugation et en éliminant le culot et récupérant le surnageant de troisième centrifugation.

La fonction de l'agent détergent, notamment la mise en œuvre de saponine, est d'éviter l'agglomération des bactéries et cellules avec des composés macromoléculaires tels que notamment globules blancs ou rouges contenus dans ledit prélèvement clinique d'urines. La mise en œuvre combinée avec un broyage mécanique permet de préparer les extraits protéiques sans mise en œuvre d'acide formique tant pour la lyse que pour la solubilisation des échantillons. Plus particulièrement encore, les volumes de prélèvements cliniques d'urine sont inférieurs à 3 mL, de préférence de pas plus de 1mL, et les concentrations de bactéries desdits prélèvements d'urines des spectres de référence et prélèvement d'urine à analyser sont de 10⁴ à 10⁷ bactéries par millilitre et les extraits protéiques peuvent être préparés à partir de pas plus de 1ml de prélèvements.

Divulguée ici est également une banque de spectres de masse de type MALDI-TOF utile comme dits spectres de référence dans un procédé selon l'invention et tel que défini ci-dessus caractérisé en ce qu'elle comprend au moins 500 dits spectres de référence, les dits spectres de référence étant réalisés sur des échantillons d'extraits protéiques obtenus selon un même dit procédé de préparation à partir d'au moins 500 prélèvements cliniques de référence de même nature, de préférence au moins 1000 prélèvements de référence, comprenant un traitement de lyse desdits microbes, sans isolement et/ou sans culture desdits microbes desdits prélèvements cliniques de référence.

Plus particulièrement, ladite banque contient des spectres de référence d'extraits protéiques de prélèvements d'urines de référence contenant au total au moins 10 dits microbes de référence, de préférence au moins 20 dits microbes de référence différents, de préférence encore au moins 40 dits microbes différents.

D'autres caractéristiques et avantages de la présente invention ressortiront mieux à la lecture de la description qui va suivre, faite de manière illustrative et non limitative, en référence aux dessins annexés sur lesquels :
- la figure 1 représente un spectre de masse MALDI-TOF d'extrait protéique d'urine stérile (non infectée),
- les figures 2A et 2B représentent des spectres de masse MALDI-TOF d'extraits protéiques à partir de colonie *d'Escherichia coli* (figure 2A) et d'urine infectée *d'Escherichia coli* (figure 2B),
- les figures 3A et 3B représentent des spectres de masse MALDI-TOF d'extraits protéiques à partir de colonie de *Klebsiella pneumoniae* (figure 3A) et d'urine infectée de *Klebsiella pneumoniae* (figure 3B),
- les figures 4A et 4B représentent des spectres de masse MALDI-TOF d'extraits protéiques à partir de colonie de *Enterococcus faecalis* (figure 4A) et d'urine infectée de *Enterococcus faecalis* (figure 4B),
- les figures 5A et 5B représentent des spectres de masse MALDI-TOF d'extraits protéiques à partir de colonie de *Proteus mirabilis* (figure 5A) et d'urine infectée *de Proteus mirabilis* (figure 5B),
- les figures 6A et 6B représentent des spectres de masse MALDI-TOF d'extraits protéiques à partir de colonie de *Pseudomonas aeruginosa* (figure 6A) et d'urine infectée de *Pseudomonosas aeruginosa* (figure 6B),
- les figures 7A et 7B représentent des spectres de masse MALDI-TOF d'extraits protéiques à partir de prélèvements d'urines infectée *d'Escherichia coli* contenant pas plus de 10 globules blancs et/ou rouge/ul (figure 2A) et plus de 10 globules blancs et/ou rouge/ul (figure 2B).

L'invention est illustrée ci-après par la description détaillée de l'identification de microorganismes par spectrométrie de masse de type MALDI-TOF directement à partir de prélèvements urinaires de patients infectés, réalisée de manière optimale non pas avec les spectres de microorganismes obtenus à partir de colonies isolées sur milieu de culture solide mais grâce à une comparaison avec les spectres obtenus à partir de prélèvements urinaires de patients présentant une infection urinaire.

L'urine est l'un des liquides biologiques produits par l'homme, constituant la plus grande part des déchets liquides du métabolisme de l'organisme. Le composant principal de l'urine est l'eau et le principal déchet qu'elle contient est l'urée mais en réalité, l'urine est un biofluide complexe avec une immense diversité chimique. Récemment, il a été mis en évidence que l'urine pouvait contenir plus de 3.000 différents composés chimiques. La complexité de la composition urinaire et le fait que les bactéries aient un métabolisme différent impliquant une expression différente des protéines selon que les bactéries soient sous forme de colonies dans un milieu de culture solide ou dans l'urine du patient infecté permettent d'expliquer les mauvais résultats quand on compare un spectre d'urine infectée par un microorganisme au spectre de ce même microorganisme isolé à l'état pur sous forme de colonies dans un milieu de culture solide.

Selon la présente invention, on a découvert qu'il était possible grâce à la création d'une base de données de spectres de prélèvements d'urines infectées de réaliser le diagnostic étiologique d'une infection urinaire par spectrométrie de masse de type MALDI-TOF avec une excellente sensibilité et spécificité comparé aux technique de culture. La culture est actuellement la technique de référence pour réaliser le diagnostic des infections urinaires. Cette technique demande au minimum de 24 à 48 h avant de pouvoir mettre en évidence le microorganisme pathogène. Grâce à la banque de données de spectres de références réalisés sur des extraits de prélèvements urinaires, la mise en évidence du microorganisme pathogène par analyse directe de l'urine par spectrométrie de masse de type MALDI-TOF nécessite moins d'1 h. La prise en charge spécifique du patient avec un traitement adapté peut donc être réalisée beaucoup plus tôt par rapport à une analyse nécessitant la culture. Le délai de prise en charge adapté est capital dans le pronostic des maladies infectieuses avec un impact direct sur la mortalité, la morbidité et le coût global du traitement.

### A) Matériel et méthodes pour la préparation de l'extrait protéique et la réalisation du spectre de masse.

### 1. Solutions et réactifs

HCCA : acide alpha cyanocinnamique, Sigma Aldrich, référence C2020-10g
TFA : Acide trifluoroacétique (Trifluoro acetic acid), Sigma Aldrich, référence 302031-100mL
Saponine : saponine, Sigma Aldrich, référence 84510-100g
Acétonitrile : qualité HPLC, VWR, référence 20060.320
Eau : qualité HPLC, VWR, référence 23595.294
Microbilles en verre : ≤ 106 µm, Sigma Aldrich, référence G4649-500g

### 2. Préparation de la solution de matrice (HCCA saturée)

- Dans un tube polypropylène 1.5 mL, introduire 2 pointes de spatules d'alpha-cyano-4-hydroxycinnamic acid (HCCA),
- Ajouter 500 µl d'acétonitrile HPLC,
- Ajouter 475 µl d'eau HPLC,
- Ajouter 25 µl de TFA (Acide trifluoroacétique),
- Vortexer ou secouer vigoureusement,
- Soniquer 10 min puis centrifuger 5 min à 13000 g,
- Transférer le surnageant dans un tube polypropylène 1.5 ml propre.

### 3. Protocole de préparation d'extrait protéique à partir d'urine.

Un ml d'urine est prélevé et vigoureusement mélangé à une solution de 300 µL de saponine dans un tube polypropylène à vis de 2 mL à l'aide d'un vortex. Après centrifugation (13000 g, 5 minutes), le culot obtenu subit un lavage avec 500 µL d'une solution d'acide trifluoroacétique 10%. Le tube est vortexé puis le mélange est centrifugé (13000 g, 5 minutes). Le culot est lavé avec 1 mL d'eau HPLC puis le tube est de nouveau vortexé et une centrifugation (13000 g, 5 minutes) est réalisée. Le surnageant doit bien être enlevé. Une spatule de microbilles en verre et 40 µL d'un mélange 50% eau / 50% acétonitrile sont ajoutés au culot obtenu. Les bactéries contenues dans le culot sont broyées avec un appareil broyeur à microbilles FastPrep®-24 (MP Biomedicals, référence 116004500) selon un programme de 4 cycles de 20 secondes puis le mélange est centrifugé (13000 g, 5 minutes). Un µL du surnageant est déposé sur la plaque cible du spectromètre puis séché 10 minutes. Un µl de solution de matrice HCCA saturée est ajouté par-dessus, séché 10 minutes, puis un spectre de masse de l'extrait protéique est réalisé sur un Microflex™ LT (Bruker).

4. La méthode d'acquisition des spectres comprend les étapes et caractéristiques suivantes :
- introduire la plaque (cible Microflex modèle de référence 224989 dénommé MSP 96) contenant les dépôts dans le spectromètre de masse Microflex LT,
- démarrer l'ordinateur associé au spectromètre pour lancer les analyses, et
- lancer l'acquisition des spectres de masse MALDI-TOF.

Les paramètres appliqués au spectromètre de masse sont les suivants :
- mode linéaire positif, Electrode IS1 : 20,00 kV, Electrode IS2 : 18,05 kV, Electrode de focalisation : 6 kV, Fréquence du laser : 60Hz, Gain du détecteur : 8,8 X, Post-ion-extraction (PIE) : 120 ns, Gamme de masse : de 700 à 20000 Da.

Les spectres sont obtenus via une méthode automatique contrôlée par le logiciel FLEXCONTROL® du fabricant BRUKER DALTONICS® les paramètres sont les suivants pour chaque spot :
- nombres séries de 40 tirs: 6 sur des positions différentes du spot (6x40 = 240 spectres),
- afin que les tirs aient balayé l'ensemble de l'échantillon de façon homogène l'acquisition se fait sur 6 positions différentes selon une géométrie hexagonale,
- pré-tirs : 10 tirs à 40% de la puissance maximum laser servent à dessaler l'échantillon (contamination par les sels minéraux),
- puissance laser : 30 % à 45 % régulée via le logiciel FLEXCONTROL®,
- sélection du spectre : sur une gamme de masse de 2000 à 20 000 Da.

L'acquisition des spectres est au final réalisée via le logiciel BIOTYPER® du fabricant BRUKER DALTONICS® qui permet d'appliquer la méthode automatique décrite ci-dessus à une série d'échantillons en prenant en compte les critères mentionnés précédemment et un score de qualité ou score de validation qui représentent l'addition de 2 notes n1 + n2 (n1 étant fonction du nombre de pics et n2 fonction du rapport signal/bruit de fond).
- n1 est la note attribuée en fonction du nombre de pics caractéristiques du spectre n, avec : n1 = 0 pour n ≤ 8, n1 = 1 pour 8 < n ≤ 27, n1 = 2 pour 27 < n ≤ 43, n1 = 3 pour 43 < n ≤ 78, n1 = 4 pour 78 < n ≤ 86, n1 = 5 pour 86 < n ≤ 110, n1 = 6 pour 110 < n, et
- n2 est la note attribuée en fonction de la valeur du rapport signal/bruit (Rmax) du pic de plus grande intensité du spectre, avec n2 = 0 pour Rmax ≤ 6,8, n2 = 1 pour 6,8 < Rmax ≤ 26,8, n2 = 2 pour 26,8 < Rmax ≤ 52,6, n2 = 3 pour 52,6 < Rmax ≤ 208,6, n2 = 4 pour 208,2 < Rmax ≤ 398,6, n2 = 5 pour 398,6 < Rmax ≤ 1 092,2, n2 = 6 pour 1 092,2 < Rmax.

Un spectre est pris en compte si son score de validation (n1+n2) est supérieur à 2.

### 5. Méthode d'identification par le logiciel Biotyper

Le logiciel BIOTYPER® fourni par le fabricant du spectromètre de masse MALDI-TOF (Bruker Daltonics) permet de comparer un nouveau spectre avec les spectres moyens contenus dans la base de données. Le logiciel BIOTYPER® identifie des spectres inconnus par comparaison avec des spectres de référence stockés dans une base de données ou autre spectre comparatif par une analyse des pics qu'ils contiennent. Les résultats d'identification sont donnés sous forme de d'un score d'identification calculé comme décrit dans le manuel d'utilisation du logiciel et résumé ci-après.

Dans un premier temps, les deux spectres sont alignés. On assigne une erreur acceptable pour la masse des pics de chaque spectre correspondant à 800 ppm (±1 Da) et le logiciel repositionne les deux spectres (autrement dit, si l'écart entre les pics du même spectre et entre les pics de l'autre spectre est trop différent, le logiciel ne pourra pas aligner les spectres).

Ensuite, les spectres obtenus subissent le traitement suivant par le logiciel BIOTYPER® 3.0:
- une soustraction de ligne de base, afin d'éliminer le bruit de de fonds, et
- un lissage des pics par mise à 0 des intensités inférieures à un seuil donné, afin d'augmenter la résolution, puis
- une liste de masse de pics est créée.

En résumé, le score d'identification est égal à log (indice1 + incide2 + indice3), avec :
- un indice il de valeur maximale de 1000 quand l'ensemble des pics de la référence de la base de données sont trouvés dans le spectre à identifier, l'indice 1 étant de zéro si il n'y a aucune correspondance, et
- un indice i2 étant de valeur maximale de 1000, si l'ordre de classification des pics par intensité croissante du spectre à identifier est identique à l'ordre de classification des pics de la référence, et
- un indice i3 étant de valeur maximale de 1000, si les pics non attribués du spectre à identifier ne se retrouve sur aucune référence.

Ainsi, un score d'identification compris entre 0 et 1,699 permet d'exclure l'identité du spectre de l'échantillon testé avec celui du spectre de référence. Un score d'identification compris entre 1,700 et 1,999 suggère que l'échantillon testé appartient ou comprend l'élément de référence et un score d'identification supérieur à 2,0 permet une identification certaine de l'échantillon testé celui du spectre de référencer présent dans la base de données.

Pour une bactérie, l'identification est la suivante :
- de 0 à 1,69 : identification non fiable
- de 1,7 à 1,99.0 : identification possible, notamment du genre pour une bactérie
- de 2.0 à 3.0 : identification certaine, notamment du genre et de l'espèce pour une bactérie.

Les critères ci-dessus sont pris en compte à l'aide notamment du logiciel FLEX CONTROL®, BIOTYPER® RTC développés par le fabricant de spectromètre de masse Bruker Daltonics® GmbH, Leipzig, Germany de référence microflex™ LT.

Plus particulièrement encore, les pics desdits spectres sont définis par une paire de coordonnées composées d'une abscisse constituée du rapport masse/charge (m/z) de 2 000 à 20 000 Da, plus particulièrement de 3 000 à 16 000 Da, la coordonnée en ordonnée étant une intensité relative ou en unité arbitraire, et on retient de préférence dans le spectre au moins les 70 pics de plus grandes intensités. On entend ici par "intensité relative", une intensité exprimée par le rapport de (S/N=intensité du pic / intensité du bruit de fond).

### 6. Méthode de création de la base de données.

Chaque spectre de référence de la base de données correspond à une moyenne de 4 spectres acquis à partir de 4 dépôts d'un réplicat biologique. Pour tous les spectres réalisés, l'opérateur évalue si le nombre de pics est suffisant et si la résolution est assez correcte pour qu'ils soient utilisés comme nouvelle référence dans la base. La base est incrémentée sur le logiciel BIOTYPER®3.1 en utilisant la méthode Bio Typer MSP Creation Standard Method (cf MALDI BIOTYPER® User Guide).

La gamme de masse pour l'évaluation et le traitement des spectres va de 4 000 Da à 16 000 Da afin de s'affranchir de la prise en compte des pics intenses obtenus dans les basses masses moléculaires (m/z < 4 000 Da) sur les spectres d'urines infectées (cf partie Résultats ci-après), qui diminuent considérablement le score d'identification lorsqu'ils sont confrontés aux spectres de référence.

Les spectres sont lissés selon l'algorithme Savitsky-Golay (Méthode standard du logiciel Biotyper 3.1 cf. Manuel d'utilisation du Biotyper) et normalisés par la valeur du pic le plus intense. La soustraction de ligne de base est effectuée selon la méthode Multipolygon (Méthode standard du logiciel Biotyper 3.1 cf. Manuel d'utilisation du Biotyper).

### B) Résultats

### 1. Obtention de spectres d'extraits protéiques d'urines stériles et infectées

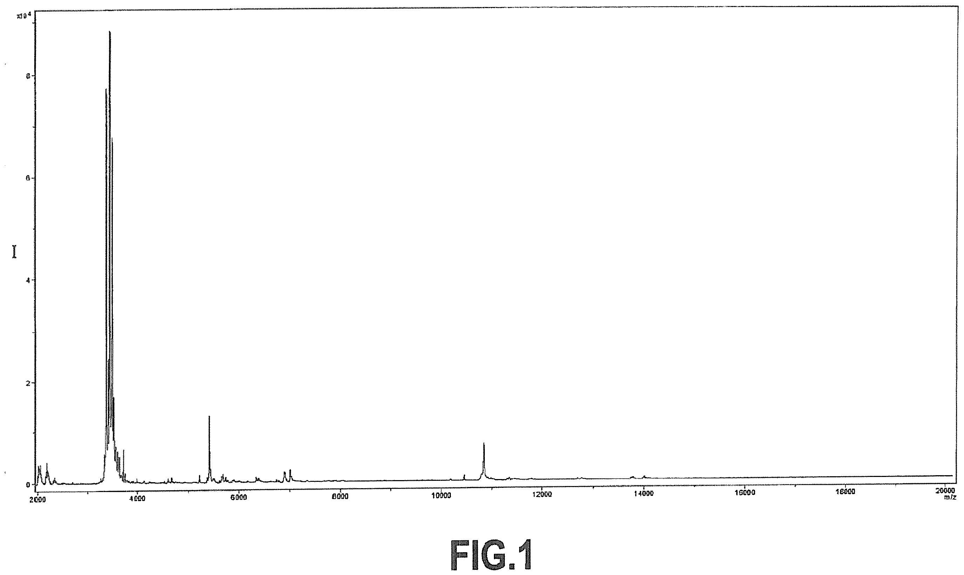

On a défini un spectre d'urines stériles. Le spectre présenté figure 1 correspond à une moyenne de spectres réalisés sur des extrait obtenus à partir d'urines stériles chez différents patients. Pour l'urine stérile, on obtient un spectre avec des pics d'origine cellulaire localisés dans les bas rapports m/z. Le triplet intense observé représente les α-défensines humaines 1, 2 et 3 (Köhling et al., Journal of Medical Microbiology (2012), 61, 339-344).

**Le tableau 1 comprend la liste des pics et la valeur de S/N pour le spectre de référence d'urine stérile selon la présente invention :**

| m/z | S/N |
|---|---|
| 2008.36 | 4.4 |
| 2022.56 | 9.5 |
| 2036.88 | 7.9 |
| 2052.11 | 9.1 |
| 2169.52 | 5.7 |
| 2185.03 | 11.0 |
| 2199.34 | 7.6 |
| 2331.89 | 3.3 |
| 3311.22 | 4.5 |
| 3326.09 | 19.4 |
| 3338.27 | 12.6 |
| 3354.99 | 30.8 |
| 3369.81 | 251.1 |
| 3404.41 | 82.3 |
| 3426.78 | 46.8 |
| 3440.96 | 288.8 |
| 3475.36 | 94.5 |
| 3485.26 | 215.8 |
| 3499.28 | 29.3 |
| 3510.93 | 57.5 |
| 3519.03 | 56.4 |
| 3554.69 | 24.3 |
| 3586.25 | 20.1 |
| 3611.98 | 15.6 |
| 3629.87 | 16.8 |
| 3708.74 | 20.0 |
| 3747.25 | 6.0 |
| 4670.37 | 3.3 |
| 5223.82 | 3.6 |
| 5381.71 | 3.3 |
| 5419.73 | 37.6 |
| 5655.66 | 3.3 |
| 5676.87 | 4.0 |
| 5736.94 | 3.1 |
| 6347.78 | 3.0 |
| 6387.80 | 3.2 |
| 6892.41 | 7.3 |
| 7006.09 | 8.8 |
| 10445.15 | 6.9 |
| 10835.89 | 52.7 |

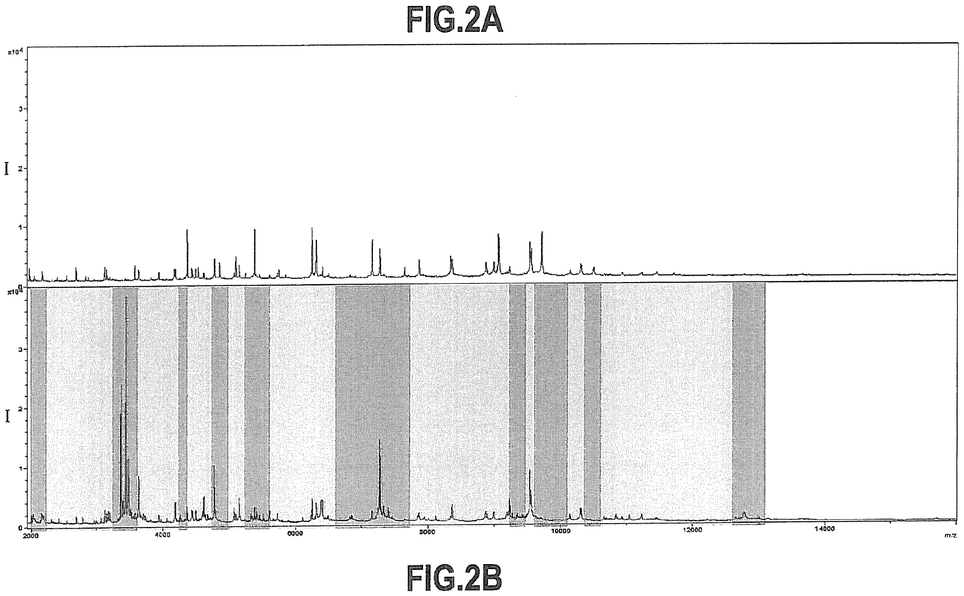

On a aussi généré les spectres d'urines infectées pour les différents microorganismes impliqués dans les infections urinaires. Sur les figures 2 à 6, à titre illustratif, on montre les résultats obtenus pour les 5 bactéries les plus fréquemment responsables d'infections urinaires. Les spectres présentés correspondent chacun à une moyenne de spectres réalisés à partir d'urines infectées par une même espèce bactérienne. Pour chaque type de bactérie analysée, les spectres à partir des colonies isolées ont également été mesurés et comparés aux spectres de la même bactérie dans les urines infectées.

On observe des profils protéiques très différents selon les conditions sous lesquelles se trouve chacune des bactéries analysées. En effet, on note des discordances telles que l'apparition ou la disparition de certains pics ainsi que des changements au niveau des intensités relatives des différents pics entre les spectres des colonies isolées et les spectres des bactéries provenant des prélèvements urinaires infectés.

Les similarités entre les spectres sont mises en évidence en gris clair et les discordances en gris foncé.

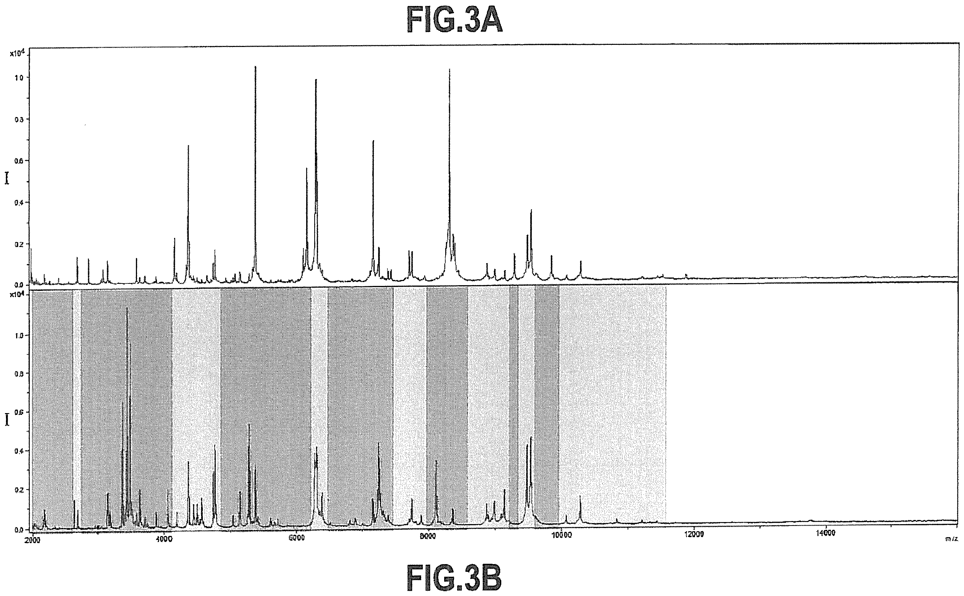

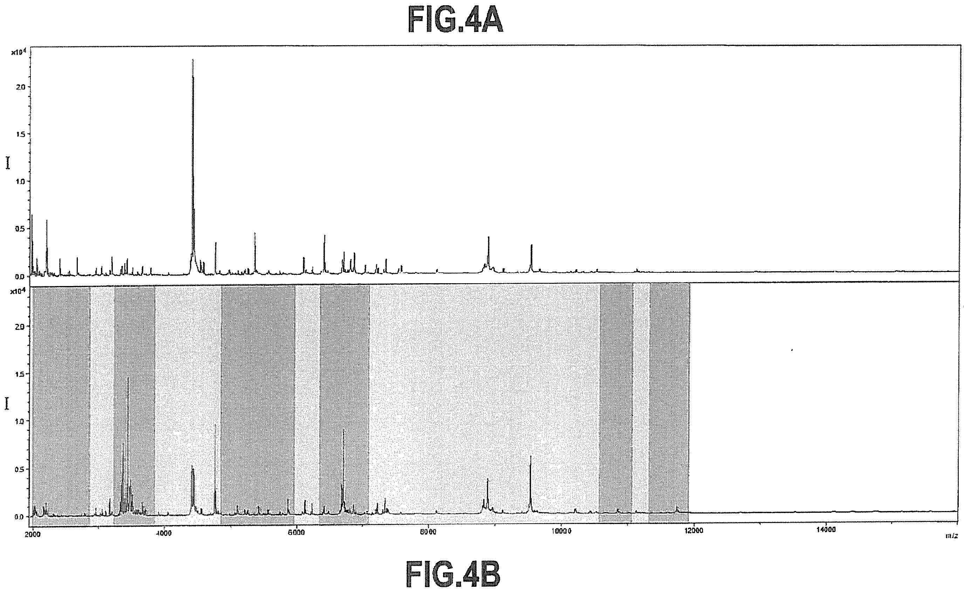

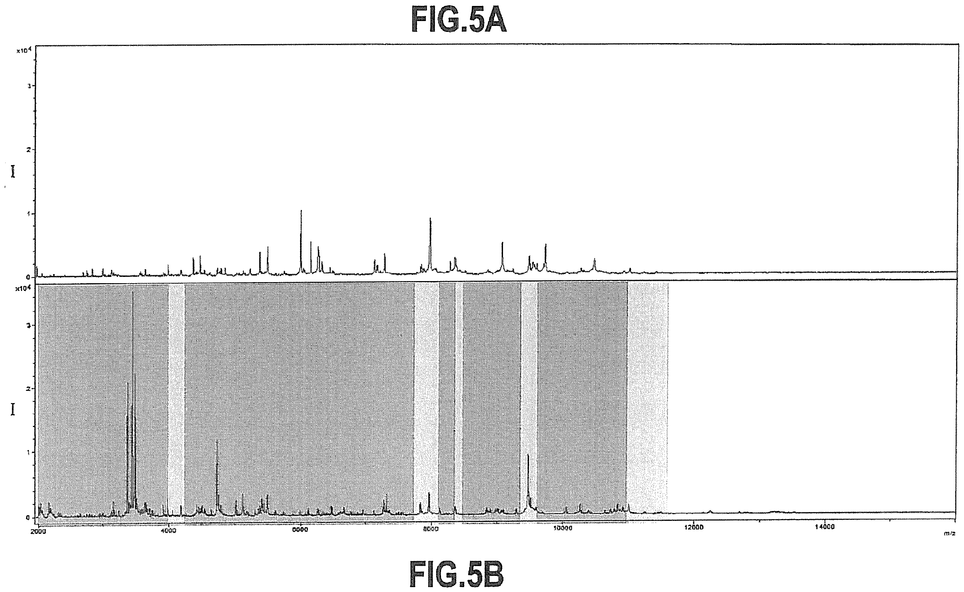

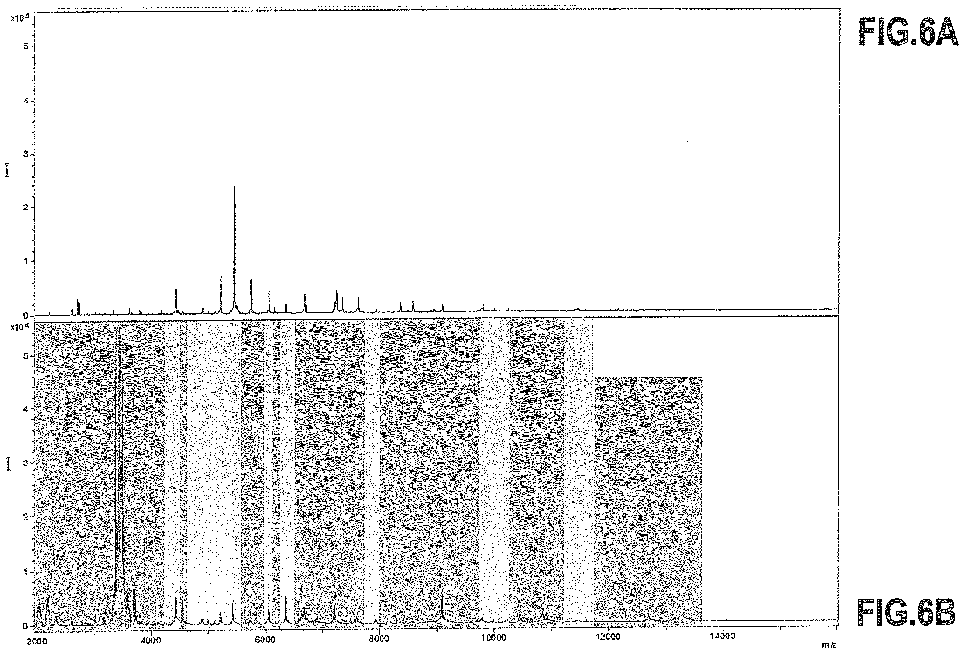

Les Fig. 2A et 2B sont des spectres obtenus pour une colonie isolée *d'Escherichia coli* et une urine infectée par *Escherichia coli.* Les fig. 3A et 3B sont des spectres obtenus pour une colonie isolée de *Klebsiella pneumoniae* et une urine infectée par *Klebsiella pneumoniae.* Les Fig. 4A et 4B sont des spectres obtenus pour une colonie isolée *d'Enterococcus faecalis* et une urine infectée par *Enterococcus faecalis.* Les fig. 5A et 5B sont des spectres obtenus pour une colonie isolée de *Proteus mirabilis* et une urine infectée par *Proteus mirabilis.* Les Fig. 6A et 6B sont des spectres obtenus pour une colonie isolée de *Pseudomonas aeruginosa* et une urine infectée par *Pseudomonas aeruginosa.*

### 2. Détection de microorganismes en extrait protéique obtenu directement à partir du prélèvement.

### A. Comparaisons avec les spectres de la base de données Biotyper de spectres d'extraits protéiques obtenus à partir de colonies de bactéries.

Les discordances observées entre les spectres des extrait obtenus à partir pour une même bactérie obtenus à partir d'une colonie de la dite bactérie et à partir de l'urine infectée par cette dernière rendent difficiles l'identification du microorganisme responsable de l'infection lorsque ces spectres sont confrontés aux spectres de référence fournis dans le logiciel BIOTYPER®, qui sont réalisés à partir de bactéries isolées.

Les spectres obtenus à partir de prélèvements urinaires de patients ont d'abord été analysés avec le logiciel BIOTYPER® RTC.

Cette analyse ne permet pas de s'affranchir des pics provenant de l'urine puisque l'ensemble du spectre est évalué. Ces pics intenses, notamment ceux causés par la présence des défensines, sont alors pris en compte dans le calcul du score d'identification et diminuent sa valeur de façon notable. En outre, le niveau d'expression des protéines par la bactérie dans l'urine infectée et la même bactérie après incubation sur gélose à 37°C n'est pas le même, ce qui génère des profils différentiels entre les spectres de la même bactérie selon les conditions dans lesquelles elle se trouve. Ces différences expliquent l'incapacité à identifier les bactéries dans les urines avec la base de données BIOTYPER® dans la majorité des cas.

### B. Comparaisons avec les spectres de la base de données de spectres d'extraits protéiques obtenus à partir d'urines infectées.

La création d'une base de données comprenant des spectres d'extraits protéiques obtenus à partir d'urines naturellement infectées a été réalisée. Cette base présente plusieurs avantages :
(1) La possibilité de décaler systématiquement le cadre d'évaluation du spectre pour la comparaison à des spectres de référence afin de ne pas avoir à prendre en compte certains pics intenses problématiques comme ceux des défensines.
(2) La prise en compte des éléments autres que bactériens générés par l'infection, tels qu'un nombre élevé de globules blancs et de globules rouges.

Ces différents avantages permettent d'obtenir une meilleure identification des microorganismes responsables des infections avec le logiciel BIOTYPER®3.1 puisqu'on confronte alors des spectres à analyser à d'autres spectres de référence mesurés dans des conditions semblables.

Pour créer la banque de données de spectres de références à partir de prélèvements urinaires de patients infectés, on a analysé les prélèvements urinaires de patients à la fois par spectrométrie de masse de type par MALDI-TOF et par culture. On a ainsi réalisé systématiquement en parallèle la culture de l'urine selon le protocole de routine du laboratoire afin de pouvoir identifier le microorganisme pathogène dans chacune d'elle. Une fois le microorganisme impliqué identifié grâce à la culture et la qualité du spectre obtenu à partir de l'urine infectée vérifiée, le spectre moyen de l'urine infectée est alors incrémenté dans une base de données spécifique des prélèvements d'urines (Base de données nommée ci-après Urinf). On a aussi analysé les spectres générés à partir des urines avec la banque de données BIOTYPER® de Bruker (spectres obtenus à partir des colonies isolées) afin de comparer les 2 banques de données.

Dans un premier temps, une base préliminaire de 56 spectres moyens de références obtenues à partir d'urines infectées et correspondant chacune à un réplicat biologique, a été créée.

**Tableau A - Composition de la base préliminaire créée de 56 références**

| Microorganismes | Nombre de références |
|---|---|
| *Escherichia coli* | 31 |
| *Klebsiella pneumoniae* | 10 |
| *Enterococcus faecalis* | 2 |
| *Proteus mirabilis* | 5 |
| *Pseudomonas aeruginosa* | 2 |
| *Enterobacter aerogenes* | 1 |
| *Staphylococcus aureus* | 2 |
| *Klebsiella oxytoca* | 3 |

Les prélèvements d'un groupe de 211 patients présentant tous des infections urinaires ont ensuite été analysés par MALDI-TOF à l'aide du logiciel BIOTYPER® RTC. Les spectres obtenus ont systématiquement été comparés aux spectres de référence fournis par Bruker. Les spectres ont également été confrontés à la base de 56 références afin de comparer les identifications obtenues dans chacun des cas.

Un score d'identification supérieur à 1.99 a été pris en considération pour conclure à une bonne identification comme recommandé par Bruker.

Le tableau B ci-après présente les résultats d'identification obtenus avec les spectres de référence Biotyper® et avec les spectres de référence de la base de données comprenant 56 références provenant d'urines infectées.

La moyenne du taux d'identification d'une base à l'autre passe de 45% à 64%.

**Tableau B**

| | **Nombre de cas** | **Score >1.99 Base Biotyper** | **% identification Base Biotyper** | **Score >1.99 Base 56 références** | **% identification Base 56 références** |
|---|---|---|---|---|---|
| *Escherichia coli* | 100 | 64 | 64% | 92 | 92% |
| *Klebsiella pneumoniae* | 23 | 10 | 43% | 21 | 91% |
| *Enterococcus faecalis* | 22 | 6 | 27% | 7 | 32% |
| *Pseudomonas aeruginosa* | 10 | 3 | 30% | 0 | 0% |
| *Proteus mirabilis* | 10 | 2 | 20% | 5 | 50% |
| *Citrobacter koseri* | 5 | 1 | 20% | 0 | 0% |
| *Enterobacter aerogenes* | 5 | 1 | 20% | 2 | 40% |
| *Enterobacter cloacae* | 5 | 1 | 20% | 0 | 0% |
| *Enterococcus faecium* | 5 | 1 | 20% | 0 | 0% |
| *Klebsiella oxytoca* | 6 | 3 | 50% | 4 | 67% |
| *Morgenella morganii* | 5 | 2 | 40% | 0 | 0% |
| *Staphylococcus aureus* | 5 | 1 | 20% | 3 | 60% |
| *Staphylococcus saprophyticus* | 5 | 0 | 0% | 0 | 0% |
| *Streptococcus agalactiae* | 5 | 1 | 20% | 0 | 0% |
| TOTAL | 211 | 96 | 45% | 134 | 64% |

Ensuite, la base de données Urinf a continué à être incrémentée jusqu'à 512 références obtenues à partir d'urines infectées et correspondant chacune à un réplicat biologique.

Le tableau C donne la composition de la base Urinf, qui contient 512 références.

**Tableau C**

| Microorganismes | Nombre de références |
|---|---|
| *Escherichia coli* | 117 |
| *Klebsiella pneumoniae* | 62 |
| *Enterococcus faecalis* | 61 |
| *Proteus mirabilis* | 50 |
| *Pseudomonas aeruginosa* | 29 |
| *Enterobacter cloacae* | 25 |
| *Streptococcus agalactiae* | 18 |
| *Staphylococcus epidermidis* | 9 |
| *Enterobacter aerogenes* | 12 |
| *Staphylococcus aureus* | 20 |
| *Klebsiella oxytoca* | 19 |
| *Citrobacter koseri* | 14 |
| *Morganella morganii* | 11 |
| *Staphylococcus saprophyticus* | 10 |
| *Enterococcus faecium* | 7 |
| *Citrobacter freundii* | 12 |
| *Acinetobacter pittii* | 2 |
| *Aerococcus urinae* | 1 |
| *Serratia marcescens* | 4 |
| *Staphylococcus capitis* | 2 |
| *Pseudomonas putida* | 2 |
| *Acinetobacter nosocomialis* | 1 |
| *Enterobacter kobei* | 1 |
| *Streptococcus oralis* | 1 |
| *Enterococcus gallinarum* | 1 |
| *Gardnerella vaginalis* | 3 |
| *Stenotrophomonas maltophilia* | 3 |
| *Raoultella ornithinolytica* | 2 |
| *Pseudomonas mosselii* | 1 |
| *Hafnia alvei* | 2 |
| *Providencia stuartii* | 1 |
| *Corynebacterium amycolatum* | 1 |
| *Corynebacterium striatum* | 1 |
| *Streptococcus anginosus* | 1 |
| *Acinetobacter baumannii* | 1 |
| *Candida albicans* | 2 |
| *Candida glabrata* | 1 |
| *Staphylococcus hominis ssp hominis* | 1 |

Les spectres obtenus à partir du même groupe de 211 patients ont alors été confrontés à cette nouvelle base de 512 références, appelée base Urinf, afin de comparer les identifications obtenues dans chacun des cas. L'analyse directe par spectrométrie de masse MALDI-TOF d'extraits protéiques obtenus à partir des urines infectées et la base de données Urinf permet de réaliser le diagnostic spécifique du microorganisme en cause dans 90% des cas.

Le tableau D présente les résultats d'identification des infections urinaires obtenus avec la base Biotyper®, la base préliminaire d'urines infectées comprenant 56 références et la base plus complète de 512 références (Base Urinf).

**Tableau D**

| | **Nombre de cas** | **Score >1.99 Base Biotyper** | **% identification Base Biotyper** | **Score >1.99 Base 56 références** | **% identification Base 56 références** | **Score >1.99 Base Urinf (512 réf.)** | **% identification Base Urinf (512 réf.)** |
|---|---|---|---|---|---|---|---|
| *Escherichia coli* | 100 | 64 | 64% | 92 | 92% | 96 | 96% |
| *Klebsiella pneumoniae* | 23 | 10 | 43% | 21 | 91% | 22 | 96% |
| *Enterococcus faecalis* | 22 | 6 | 27% | 7 | 32% | 19 | 86% |
| *Pseudomonas aeruginosa* | 10 | 3 | 30% | 0 | 0% | 8 | 80% |
| *Proteus mirabilis* | 10 | 2 | 20% | 5 | 50% | 7 | 70% |
| *Citrobacter koseri* | 5 | 1 | 20% | 0 | 0% | 4 | 80% |
| *Enterobocter aerogenes* | 5 | 1 | 20% | 2 | 40% | 4 | 80% |
| *Enterobacter cloacae* | 5 | 1 | 20% | 0 | 0% | 5 | 100% |
| *Enterococcus faecium* | 5 | 1 | 20% | 0 | 0% | 3 | 60% |
| *Klebsiella oxytoca* | 6 | 3 | 50% | 4 | 67% | 6 | 100% |
| *Morganella morganii* | 5 | 2 | 40% | 0 | 0% | 4 | 80% |
| *Staphylococcus aureus* | 5 | 1 | 20% | 3 | 60% | 5 | 100% |
| *Staphylococcus saprophyticus* | 5 | 0 | 0% | 0 | 0% | 3 | 60% |
| *Streptococcus agalactiae* | 5 | 1 | 20% | 0 | 0% | 4 | 80% |
| TOTAL | 211 | 96 | 45% | 134 | 64% | 190 | 90% |

Dans ce travail, pour les prélèvements urinaires infectés, les bactéries étaient présentes dans des concentrations variables de 10⁴ à 10⁷/ml, la base Urinf contenait des spectres obtenus avec des urines contenant des taux de globules blanc et/ou globules rouges variables. La présence de prélèvements urinaires avec au moins ou plus de 10 globules blancs et/ou globules rouges par µl n'a pas empêché l'identification bactérienne. Et, même, pour les patients ne présentant que très peu de globules rouges et de globules blancs (< 10 / µl) dans leurs urines, l'identification bactérienne n'a pas été affectée. On avait donc toujours :
- un premier groupe de prélèvements d'urines contenant pas plus de 10 globules blancs et/ou de globules rouges /µl correspondant aux patients avec une infection urinaire et une faible réponse inflammatoire locale, et
- un deuxième groupe de prélèvements d'urines contenant plus de 10 globules blancs et/ou de globules rouge /µl, correspondant aux patients avec une infection urinaire et une réponse inflammatoire locale élevée.

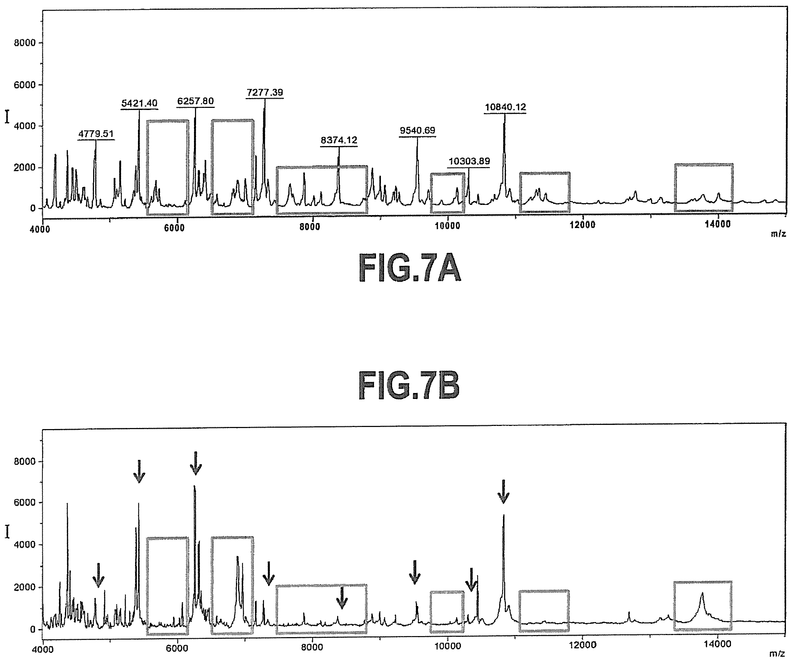

Sur les Figures 7A et 7B on compare des spectres d'urines infectées par *E. coli* avec des différences quantitatives en globules blancs et globules rouges. Le spectre de la figure 7A a été réalisé à partir de prélèvement d'urines contenant 6 globules blancs par µl et 4 globules rouges par µl. Le spectre de la figure 7B a été réalisé à partir de prélèvement d'urines contenant 7957 globules blancs par µl et 24734 globules rouges par µl. Des pics discriminants *d'E. coli* sont indiqués par les valeurs m/z et les flèches. Les différences entre les spectres sont mises en évidence par les rectangles gris.

Le tableau 2 ci-après reprend la liste de 10 pics discriminants et la valeur de S/N pour le spectre d'urine infectée par *E. coli* dans les différents groupes de prélèvements :

| m/z | S/N |
|---|---|
| 4779.21 | 14.4 |
| 5152.67 | 10.6 |
| 5421.57 | 4.2 |
| 6258.86 | 11.4 |
| 6414.59 | 11.7 |
| 7277.45 | 43.0 |
| 8373.90 | 9.8 |
| 9540.65 | 35.0 |
| 10304.57 | 9.4 |
| 10840.74 | 5.1 |

Le tableau 3 ci-après reprend la liste de 10 pics discriminants et la valeur de S/N pour le spectre d'urine infectée par *K. pneumoniae* dans les différents groupes de prélèvements :

| m/z | S/N |
|---|---|
| 4364.91 | 39.2 |
| 5280.61 | 58.1 |
| 5382.02 | 36.4 |
| 6276.29 | 45.4 |
| 6299.95 | 50.5 |
| 7244.92 | 55.5 |
| 7263.64 | 39.9 |
| 8118.92 | 46.2 |
| 9477.05 | 84.7 |
| 9539.49 | 93.7 |

Le tableau 4 ci-après reprend la liste de 10 pics discriminants et la valeur de S/N pour le spectre d'urine infectée par *E. faecalis* dans les différents groupes de prélèvements :

| m/z | S/N |
|---|---|
| 4414.96 | 34.9 |
| 4429.75 | 14.7 |
| 4443.81 | 29.2 |
| 4767.53 | 52.2 |
| 6705.98 | 66.2 |
| 6722.99 | 12.1 |
| 7337.20 | 14.1 |
| 8832.96 | 17.5 |
| 8887.75 | 41.7 |
| 9534.19 | 83.4 |

Le tableau 5 ci-après reprend la liste de 10 pics discriminants et la valeur de S/N pour le spectre d'urine infectée par *P. mirabilis* dans les différents groupes de prélèvements :

| m/z | S/N |
|---|---|
| 4738.52 | 49.1 |
| 4757.88 | 14.8 |
| 5132.81 | 14.3 |
| 5421.33 | 11.0 |
| 5504.49 | 15.3 |
| 7278.05 | 12.5 |
| 7312.52 | 17.7 |
| 7967.22 | 20.7 |
| 9476.91 | 75.6 |
| 9516.21 | 22.1 |

Le tableau 6 ci-après reprend la liste de 10 pics discriminants et la valeur de S/N pour le spectre d'urine infectée par *P. aeruginosa* dans les différents groupes de prélèvements :

| m/z | S/N |
|---|---|
| 4436.62 | 12.6 |
| 4545.92 | 10.4 |
| 5212.32 | 5.4 |
| 5421.64 | 9.6 |
| 6049.26 | 13.3 |
| 6350.33 | 12.9 |
| 6640.83 | 5.4 |
| 7206.22 | 10.5 |
| 9091.90 | 25.3 |
| 10841.01 | 20.4 |

Le tableau 7 ci-après reprend la liste de 10 pics discriminants et la valeur de S/N pour le spectre d'urine infectée par *C. koseri* dans les différents groupes de prélèvements :

| m/z | S/N |
|---|---|
| 4599.25 | 17.5 |
| 4762.90 | 26.0 |
| 5151.83 | 4.6 |
| 6257.60 | 10.5 |
| 6385.74 | 8.6 |
| 7275.76 | 11.1 |
| 7388.54 | 9.1 |
| 9199.12 | 21.8 |
| 9525.35 | 44.1 |
| 10302.31 | 5.6 |

Le tableau 8 ci-après reprend la liste de 10 pics discriminants et la valeur de S/N pour le spectre d'urine infectée par *E. aerogenes* dans les différents groupes de prélèvements :

| m/z | S/N |
|---|---|
| 4571.20 | 7.8 |
| 4741.77 | 6.2 |
| 5422.08 | 17.9 |
| 6293.90 | 6.5 |
| 7249.28 | 8.8 |
| 7277.31 | 10.5 |
| 7652.80 | 11.8 |
| 9143.52 | 10.2 |
| 9484.08 | 10.7 |
| 10842.93 | 27.3 |

Le tableau 9 ci-après reprend la liste de 10 pics discriminants et la valeur de S/N pour le spectre d'urine infectée par *E. cloacae* dans les différents groupes de prélèvements :

| m/z | S/N |
|---|---|
| 4755.06 | 20.1 |
| 9510.14 | 32.5 |
| 7247.02 | 14.1 |
| 4765.80 | 9.8 |
| 7275.50 | 9.8 |
| 4571.08 | 7.8 |
| 6258.64 | 8.2 |
| 9142.46 | 11.0 |
| 5152.86 | 5.3 |
| 6462.65 | 3.1 |

Le tableau 10 ci-après reprend la liste de 10 pics discriminants et la valeur de S/N pour le spectre d'urine infectée par *E. faecium* dans les différents groupes de prélèvements :

| m/z | S/N |
|---|---|
| 4779.51 | 7.5 |
| 5358.95 | 4.4 |
| 5870.87 | 9.1 |
| 7293.84 | 6.7 |
| 7350.11 | 6.5 |
| 7689.87 | 4.8 |
| 9066.66 | 3.8 |
| 9558.48 | 15.5 |
| 10079.17 | 6.1 |
| 11736.83 | 19.4 |

Le tableau 11 ci-après reprend la liste de 10 pics discriminants et la valeur de S/N pour le spectre d'urine infectée par *K. oxytoca* dans les différents groupes de prélèvements :

| m/z | S/N |
|---|---|
| 4187.06 | 6.6 |
| 4366.29 | 8.2 |
| 4734.96 | 13.9 |
| 4780.95 | 13.7 |
| 5193.49 | 4.7 |
| 6259.92 | 16.0 |
| 7249.63 | 17.2 |
| 9144.54 | 10.4 |
| 9469.89 | 26.4 |
| 9561.48 | 21.6 |

Le tableau 12 ci-après reprend la liste de 10 pics discriminants et la valeur de S/N pour le spectre d'urine infectée par *M. morganii* dans les différents groupes de prélèvements :

| m/z | S/N |
|---|---|
| 4373.45 | 7.4 |
| 4641.58 | 3.7 |
| 4733.32 | 20.0 |
| 4745.13 | 4.4 |
| 5138.48 | 7.3 |
| 6746.57 | 10.4 |
| 7276.36 | 12.8 |
| 9282.83 | 5.2 |
| 9466.05 | 31.3 |
| 10274.90 | 8.4 |

Le tableau 13 ci-après reprend la liste de 10 pics discriminants et la valeur de S/N pour le spectre d'urine infectée par *S.aureus* dans les différents groupes de prélèvements :

| m/z | S/N |
|---|---|
| 4813.30 | 15.4 |
| 4827.03 | 8.3 |
| 6423.98 | 13.3 |
| 6847.15 | 5.0 |
| 7286.97 | 3.6 |
| 8091.23 | 6.6 |
| 8891.08 | 12.8 |
| 9626.23 | 31.0 |
| 9653.69 | 14.3 |
| 10103.41 | 6.7 |

Le tableau 14 ci-après reprend la liste de 5 pics discriminants et la valeur de S/N pour le spectre d'urine infectée par *S. saprophyticus* dans les différents groupes de prélèvements :

| m/z | S/N |
|---|---|
| 6617.70 | 3.6 |
| 6860.33 | 3.9 |
| 9048.93 | 3.1 |
| 9640.48 | 3.3 |
| 12686.88 | 9.1 |

Le tableau 15 ci-après reprend la liste de 10 pics discriminants et la valeur de S/N pour le spectre d'urine infectée par *S. agalactiae* dans les différents groupes de prélèvements :

| m/z | S/N |
|---|---|
| 4099.14 | 14.3 |
| 4450.94 | 31.7 |
| 5952.98 | 21.0 |
| 6181.43 | 19.5 |
| 6733.69 | 30.8 |
| 6811.08 | 40.9 |
| 6842.29 | 17.7 |
| 6938.25 | 44.6 |
| 8199.11 | 32.2 |
| 9457.24 | 3.7 |

La base de spectres d'urines (« base Urinf ») a été complétée régulièrement et compte à ce jour 1000 spectres de références réalisés à partir de 1000 échantillons d'urines comprenant 40 espèces de bactéries différentes. Cette base sera encore enrichie graduellement à l'avenir. La composition de la base actuelle est donnée dans le tableau E.

**Tableau E**

| **Microorganismes** | **Nombre de références** |
|---|---|
| *Escherichia coli* | 152 |
| *Klebsiella pneumoniae* | 80 |
| *Enterococcus faecalis* | 85 |
| *Proteus mirabilis* | 95 |
| *Pseudomonas aeruginosa* | 76 |
| *Enterobacter cloacae* | 80 |
| *Streptococcus agalactiae* | 43 |
| *Staphylococcus epidermidis* | 30 |
| *Enterobacter aerogenes* | 36 |
| *Staphylococcus aureus* | 44 |
| *Klebsiella oxytoca* | 31 |
| *Citrobacter koseri* | 44 |
| *Morganella morganii* | 24 |
| *Staphylococcus saprophyticus* | 37 |
| *Enterococcus faecium* | 29 |
| *Citrobacter freundii* | 20 |
| *Serratia marcescens* | 15 |
| *Gardnerella vaginalis* | 6 |
| *Stenotrophomonas maltophilia* | 4 |
| *Staphylococcus capitis* | 2 |
| *Staphylococcus hominis* | 3 |
| *Staphylococcus haemolyticus* | 4 |
| *Pseudomonas putida* | 3 |
| *Pseudomonas mosselii* | 1 |
| *Pseudomonas plecoglossicida* | 1 |
| *Streptococcus oralis* | 4 |
| *Streptococcus anginosus* | 2 |
| *Streptococcus mitis* | 2 |
| *Streptococcus pneumoniae* | 1 |
| *Aerococcus urinae* | 3 |
| *Proteus vulgaris* | 5 |
| *Acinetobacter pittii* | 4 |
| *Acinetobacter nosocomialis* | 1 |
| *Acinetobacter baumannii* | 3 |
| *Hafnia alvei* | 4 |
| *Providencia stuartii* | 4 |
| *Providencia rettgeri* | 1 |
| *Corynebacterium amycolatum* | 1 |
| *Corynebacterium striatum* | 1 |
| *Corynebacterium tuberculostearicum* | 1 |
| *Enterococcus gallinarum* | 2 |
| *Enterobacter asburiae* | 2 |
| *Enterobacter kobei* | 3 |
| *Enterobacter ludwigii* | 1 |
| *Raoultella ornithinolytica* | 3 |
| *Citrobacter amalonaticus* | 3 |
| *Citrobacter braakii* | 1 |
| *Candida albicans* | 2 |
| *Candida glabrata* | 1 |

Les résultats obtenus par la création d'une banque de données spécifiques d'urines infectées nous permettent de :
- Détecter sans étape d'isolement la présence de microorganismes dans les prélèvements de patients tels que les urines avec une excellente sensibilité ; et
- Identifier le microorganisme en cause.

## Revendications

1. Procédé d'identification d'un microbe dans un prélèvement clinique par comparaison de spectres de masse réalisés par spectrométrie de masse de type MALDI-TOF d'un échantillon d'extrait protéique du dit prélèvement clinique à analyser contenant un dit microbe, par comparaison avec des spectres de référence d'au moins une banque de spectres d'une pluralité d'extraits protéiques d'échantillons contenant chacun un microbe choisi parmi respectivement une pluralité de microbes de référence,
**caractérisé en ce qu'**on réalise les étapes dans lesquelles :
a) on établit une banque de spectres de référence par spectrométrie de masse de type MALDI-TOF, lesdits spectres de référence étant réalisés sur des échantillons de référence d'extraits protéiques obtenus selon un même procédé de préparation à partir de prélèvements cliniques de référence de même nature comprenant un traitement de lyse des microbes, sans isolement et/ou sans culture desdits microbes desdits prélèvements cliniques de référence, et
b) on réalise un spectre d'un échantillon à analyser d'extrait protéique obtenu à partir d'un prélèvement clinique à analyser de même nature que celui des échantillons de référence, ledit échantillon à analyser étant obtenu selon le même dit procédé de préparation à partir dudit prélèvement clinique comprenant un traitement de lyse des microbes éventuels qu'il est susceptible de contenir, sans isolement et/ou sans culture dudit microbe dudit prélèvement clinique à analyser, et
c) on détermine que ledit prélèvement clinique à partir duquel un échantillon à analyser a été obtenu, contient un microbe de référence donné choisi parmi ladite pluralité de différents microbes de référence de ladite banque de référence si on retrouve dans le spectre de l'échantillon à analyser de l'étape b) l'ensemble des pics caractéristiques d'un dit spectre de référence d'un échantillon de référence contenant ledit microbe de référence donné.

2. Procédé selon la revendication 1 **caractérisé en ce que** ladite nature desdits prélèvements cliniques consiste dans des urine, liquide céphalo-rachidien, lavage broncho-alvéolaire ou sang.

3. Procédé selon la revendication 1 ou 2 **caractérisé en ce que**, à l'étape a) pour chaque microbe de référence, la banque comprend une pluralité de spectres réalisés sur une pluralité d'échantillons obtenus à partir de respectivement une pluralité de catégories distinctes de prélèvements cliniques d'une même nature de prélèvement clinique, les différentes catégories distinctes de prélèvements cliniques d'une même nature pour un même microbe de référence correspondant de préférence à des prélèvements cliniques dont les composants autres que le microbe au sein dudit prélèvement clinique sont qualitativement et/ou quantitativement différents induisant des différences spectrales de tout ou partie de pics caractéristiques des protéines ou fragments de protéines provenant dudit microbe de référence.

4. Procédé selon la revendication 3 **caractérisé en ce que** les dits spectres de référence sont obtenus à partir de différentes catégories distinctes de prélèvements d'urines comprenant des concentrations en globules blancs et/ou globules rouges différentes, avec de préférence au moins :
- un premier groupe de prélèvements d'urines contenant pas plus de 10 globules blancs et/ou de globules rouges /µl, et
- un deuxième groupe de prélèvements d'urines contenant plus de 10 globules blancs et/ou de globules rouge /µl.

5. Procédé selon l'une des revendications 1 à 4 **caractérisé en ce que** lesdits microbes de référence sont choisis parmi les bactéries et les levures, de préférence les bactéries.

6. Procédé selon la revendication 5 **caractérisé en ce que**, pour des prélèvements d'urines, lesdites bactéries de référence comprennent au moins *Escherichia coli, Klebsiella pneumoniae, Enterococcus faecalis, Proteus mirabilis, Pseudomonas aeruginosa.*

7. Procédé selon la revendication 5 ou 6 **caractérisé en ce que** lesdites bactéries de référence sont choisies parmi au moins *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii.*

8. Procédé selon l'une des revendications 1 à 7 **caractérisé en ce que** lesdits microbes de référence sont choisis parmi au moins : *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii, Pseudomonas putida Serratia marcescens*, *Klebsiella oxytoca, Aerococcus urinae, Staphylococcus capitis ; Pseudomonas putida, Acinetobacter nosocomialis, Enterobacter kobei, Streptococcus oralis, Enterococcus gallinarum, Stenotrophomonas maltophilia, Raoultella ornithinolytica, Pseudomonas mosselii, Hafnia alvei, Providencia stuartii, Corynebacterium amycolatum, Corynebacterium striatum, Streptococcus anginosus, Acinetobacter baumannii, Staphylococcus hominis ssp hominis, Gardnerella vaginalis, Candida albicans et Candida glabrata.*

9. Procédé selon l'une des revendications 1 à 8 **caractérisé en ce que** ladite banque de référence comprend en outre au moins un spectre de référence d'extrait protéique de dit prélèvement clinique sain non infecté par un microbe.

10. Procédé selon l'une des revendications 1 à 9 **caractérisé en ce que** ladite banque de référence comprend au moins 500 spectres de référence réalisés à partir d'au moins 500 échantillons de dits prélèvements cliniques contenant chacun un dit microbe de référence de préférence l'ensemble desdits prélèvements cliniques de référence contenant au moins 10 dits microbes de référence différents, de préférence au moins 20 dits microbes de référence différents.

11. Procédé selon l'une des revendications 1 à 10 **caractérisé en ce que** lesdits extraits protéiques sont obtenus en effectuant un procédé de préparation comprenant les étapes successives suivantes :
1) on élimine le liquide du dit prélèvement clinique à analyser, et on récupère un premier résidu solide, et
2) de préférence, on ajoute une solution de détergent au dit premier résidu solide, et
3) on élimine le liquide à nouveau et on récupère un deuxième résidu solide, et
4) on réalise une lyse des cellules présente dans ledit deuxième résidu solide, de préférence une lyse mécanique, et
5) on solubilise les protéines et fragments de protéines présents dans le dit deuxième résidu solide, avec une solution de solvant, et
6) on élimine les substances non solubilisées.

12. Procédé selon la revendication 11 **caractérisé en ce que** lesdits extraits protéiques sont obtenus en effectuant un procédé de préparation comprenant les étapes successives suivantes:
1) on élimine le liquide du dit prélèvement clinique à analyser, en réalisant une première centrifugation et éliminant le surnageant de centrifugation, et en récupérant un premier résidu solide qui est le culot de première centrifugation, et
2) on ajoute une solution de détergent au dit premier résidu solide, de préférence une solution de saponine, et
3) on récupère un deuxième résidu solide par une deuxième centrifugation et en éliminant le surnageant et en récupérant un deuxième résidu solide qui est le culot de deuxième centrifugation, et
4) on réalise une lyse mécanique dudit deuxième résidu solide par broyage mécanique permettant d'obtenir des fragments de taille inférieure ou égale à 1 µm de préférence réalisé à l'aide de microbilles de verre, et
5) on solubilise les protéines et fragments de protéines présents dans le dit deuxième résidu solide de lysat cellulaire, de préférence avec une solution d'acide trifluoro-acétique et acétonitrile, et
6) on élimine les substances non solubilisées, par une troisième centrifugation et en éliminant le culot et récupérant le surnageant de troisième centrifugation.

13. Procédé selon l'une des revendications 1 à 12 **caractérisé en ce qu'**on utilise des volumes de prélèvements cliniques d'urine inférieurs à 3 mL de préférence pas plus de 1mL et les concentrations de bactéries desdits prélèvements d'urines des spectres de référence et dit prélèvement d'urine à analyser sont de 10⁴ à 10⁷ bactéries par millilitre.

## Patentansprüche

1. Verfahren zum Identifizieren einer Mikrobe in einer klinischen Probe durch Vergleich von Massenspektren, die durch Massenspektrometrie vom Typ MALDI-TOF einer Proteinextraktprobe der zu analysierenden, die Mikrobe enthaltenden klinischen Probe erzeugt wurden, mit Referenzspektren mindestens einer Bibliothek von Spektren einer Mehrzahl von Proteinextrakten von Proben, die jeweils eine aus einer entsprechenden Mehrzahl von Referenzmikroben ausgewählte Mikrobe enthalten, **dadurch gekennzeichnet, dass** die Schritte durchgeführt werden, in denen:
a) eine Bibliothek von Referenzspektren durch Massenspektrometrie vom Typ MALDI-TOF erstellt wird, wobei die Referenzspektren an Referenzproben von Proteinextrakten erzeugt werden, die nach dem gleichen Herstellungsverfahren aus klinischen Referenzproben gleicher Art erhalten werden, umfassend eine Lysebehandlung der Mikroben, ohne Isolierung und/oder ohne Kultur der Mikroben aus den klinischen Referenzproben, und
b) ein Spektrum einer zu analysierenden Proteinextraktprobe erzeugt wird, die aus einer zu analysierenden klinischen Probe der gleichen Art wie die der Referenzproben erhalten wird, wobei die zu analysierende Probe nach dem gleichen Herstellungsverfahren aus der klinischen Probe erhalten wird, umfassend eine Lysebehandlung von eventuell darin enthaltenen Mikroben, ohne Isolierung und/oder ohne Kultur der Mikrobe aus der zu analysierenden klinischen Probe, und
c) bestimmt wird, dass die klinische Probe, aus der eine zu analysierende Probe erhalten wurde, eine gegebene Referenzmikrobe enthält, die aus der Mehrzahl von verschiedenen Referenzmikroben der Referenzbibliothek ausgewählt wurde, wenn im Spektrum der zu analysierenden Probe von Schritt b) alle charakteristischen Peaks eines solchen Referenzspektrums einer Referenzprobe, die die gegebene Referenzmikrobe enthält, zu finden sind.

2. Verfahren nach Anspruch 1, **dadurch gekennzeichnet, dass** die Art der klinischen Proben aus Urin, Zerebrospinalflüssigkeit, bronchoalveolärer Lavage oder Blut besteht.

3. Verfahren nach Anspruch 1 oder 2, **dadurch gekennzeichnet, dass** in Schritt a) für jede Referenzmikrobe die Bibliothek eine Mehrzahl von Spektren umfasst, die an einer Mehrzahl von Proben erzeugt wurden, die aus jeweils einer Mehrzahl von unterschiedlichen Kategorien von klinischen Proben derselben Art von klinischen Proben erhalten wurden, wobei die verschiedenen unterschiedlichen Kategorien von klinischen Proben derselben Art für dieselbe Referenzmikrobe vorzugsweise klinischen Proben entsprechen, deren andere Komponenten als die Mikrobe innerhalb der klinischen Probe qualitativ und/oder quantitativ unterschiedlich sind, wodurch spektrale Unterschiede aller oder eines Teils der charakteristischen Peaks der Proteine oder Proteinfragmente von der Referenzmikrobe induziert werden.

4. Verfahren nach Anspruch 3, **dadurch gekennzeichnet, dass** die Referenzspektren aus verschiedenen unterschiedlichen Kategorien von Urinproben erhalten werden, die unterschiedliche Konzentrationen von weißen Blutkörperchen und/oder roten Blutkörperchen umfassen, vorzugsweise mit mindestens:
- einer ersten Gruppe von Urinproben, die nicht mehr als 10 weiße Blutkörperchen und/oder rote Blutkörperchen /µl enthalten, und
- einer zweiten Gruppe von Urinproben, die mehr als 10 weiße Blutkörperchen und/oder rote Blutkörperchen /µl enthalten.

5. Verfahren nach einem der Ansprüche 1 bis 4, **dadurch gekennzeichnet, dass** die Referenzmikroben aus Bakterien und Hefen, vorzugsweise Bakterien, ausgewählt werden.

6. Verfahren nach Anspruch 5, **dadurch gekennzeichnet, dass** die Referenzbakterien für Urinproben mindestens *Escherichia coli, Klebsiella pneumoniae, Enterococcus faecalis, Proteus mirabilis, Pseudomonas aeruginosa* umfassen.

7. Verfahren nach Anspruch 5 oder 6, **dadurch gekennzeichnet, dass** die Referenzbakterien ausgewählt sind aus mindestens *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii.*

8. Verfahren nach einem der Ansprüche 1 bis 7, **dadurch gekennzeichnet, dass** die Referenzmikroben ausgewählt sind aus mindestens: *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii, Pseudomonas putida Serratia marcescens, Klebsiella oxytoca, Aerococcus urinae, Staphylococcus capitis ; Pseudomonas putida, Acinetobacter nosocomialis, Enterobacter kobei, Streptococcus oralis, Enterococcus gallinarum, Stenotrophomonas maltophilia, Raoultella ornithinolytica, Pseudomonas mosselii, Hafnia alvei, Providencia stuartii, Corynebacterium amycolatum, Corynebacterium striatum, Streptococcus anginosus, Acinetobacter baumannii, Staphylococcus hominis ssp hominis, Gardnerella vaginalis, Candida albicans* und *Candida glabrata.*

9. Verfahren nach einem der Ansprüche 1 bis 8, **dadurch gekennzeichnet, dass** die Referenzbibliothek ferner mindestens ein Referenzspektrum von Proteinextrakt der gesunden klinischen Probe, die nicht durch eine Mikrobe infiziert ist, umfasst.

10. Verfahren nach einem der Ansprüche 1 bis 9, **dadurch gekennzeichnet, dass** die Referenzbibliothek mindestens 500 Referenzspektren umfasst, die aus mindestens 500 Proben der klinischen Proben erzeugt wurden, die jeweils eine der Referenzmikroben enthalten, wobei vorzugsweise alle klinischen Referenzproben mindestens 10 verschiedene der Referenzmikroben, vorzugsweise mindestens 20 verschiedene der Referenzmikroben, enthalten.

11. Verfahren nach einem der Ansprüche 1 bis 10, **dadurch gekennzeichnet, dass** die Proteinextrakte durch die Durchführung eines Herstellungsverfahrens erhalten werden, das die folgenden aufeinander folgenden Schritte umfasst:
1) Entfernen der Flüssigkeit aus der zu analysierenden klinischen Probe und Gewinnen eines ersten festen Rückstands, und
2) vorzugsweise Hinzufügen einer Reinigungsmittellösung zu dem ersten festen Rückstand, und
3) erneutes Entfernen der Flüssigkeit und Gewinnen eines zweiten festen Rückstands, und
4) Durchführen einer Lyse der in diesem zweiten festen Rückstand vorhandenen Zellen, vorzugsweise eine mechanische Lyse, und
5) Solubilisieren der in diesem zweiten festen Rückstand vorhandenen Proteine und Proteinfragmente mit einer Lösungsmittellösung, und
6) Entfernen der nicht gelösten Substanzen.

12. Verfahren nach Anspruch 11, **dadurch gekennzeichnet, dass** die Proteinextrakte durch die Durchführung eines Herstellungsverfahrens erhalten werden, das die folgenden aufeinander folgenden Schritte umfasst:
1) Entfernen der Flüssigkeit aus der zu analysierenden klinischen Probe durch Durchführen einer ersten Zentrifugation und Entfernen des Zentrifugationsüberstands und Gewinnen eines ersten festen Rückstands, der das erste Zentrifugationspellet darstellt, und
2) Hinzufügen einer Reinigungsmittellösung, vorzugsweise einer Saponinlösung, zu dem ersten festen Rückstand, und
3) Gewinnen eines zweiten festen Rückstands durch eine zweite Zentrifugation und durch Entfernen des Überstands und Gewinnen eines zweiten festen Rückstands, der das zweite Zentrifugationspellet darstellt, und
4) Durchführen einer mechanischen Lyse des zweiten festen Rückstands durch mechanisches Mahlen, wodurch es möglich wird, Fragmente mit einer Größe von weniger als oder gleich 1 µm zu erhalten, vorzugsweise unter Verwendung von Glasmikrokügelchen, und
5) Solubilisieren der in diesem zweiten festen Rückstand des Zelllysats vorhandenen Proteine und Proteinfragmente, vorzugsweise mit einer Lösung aus Trifluoressigsäure und Acetonitril, und
6) Entfernen der nicht gelösten Substanzen durch eine dritte Zentrifugation und durch Entfernen des Pellets und Gewinnen des dritten Zentrifugationsüberstands.

13. Verfahren nach einem der Ansprüche 1 bis 12, **dadurch gekennzeichnet, dass** Volumina von klinischen Urinproben von weniger als 3 ml, vorzugsweise nicht mehr als 1 ml, verwendet werden und die Bakterienkonzentrationen der Urinproben der Referenzspektren und der zu analysierenden Urinprobe 10⁴ bis 10⁷ Bakterien pro Milliliter betragen.

## Claims

1. A process for identifying a microbe in a clinical sample by comparison of mass spectra produced by MALDI-TOF type mass spectrometry of a protein extract sample of said clinical sample to be analyzed containing said microbe, by comparison with reference spectra of at least one bank of spectra of a plurality of protein extracts of samples each containing a microbe chosen among respectively a plurality of reference microbes,
**characterized in that** the steps are performed in which:
a) a bank of reference spectra by MALDI-TOF type mass spectrometry is established, said reference spectra being produced on reference samples of protein extracts obtained by the same preparation process from clinical reference samples of the same nature comprising a lysis treatment of the microbes, without isolation and/or without culture of said microbes from said clinical reference samples, and
b) a spectrum of a protein extract sample to be analyzed obtained from a clinical sample to be analyzed of the same nature as that of the reference samples is produced, said sample to be analyzed being obtained according to the same said preparation process from said clinical sample comprising a lysis treatment of any microbes it may contain, without isolation and/or without culture of said microbe from said clinical sample to be analyzed, and
c) it is determined that said clinical specimen from which a sample to be analyzed has been obtained contains a given reference microbe selected from said plurality of different reference microbes of said reference library if the spectrum of the sample to be analyzed of step b) contains the set of characteristic peaks of a said reference spectrum of a reference sample containing said given reference microbe.

2. The process as claimed in claim 1 **characterized in that** said nature of said clinical samples consists of urine, cerebrospinal fluid, bronchoalveolar lavage or blood.

3. The process as claimed in claim 1 or 2 **characterized in that**, in step a) for each reference microbe, the library comprises a plurality of spectra performed on a plurality of samples obtained from respectively a plurality of distinct categories of clinical samples of the same nature of clinical specimen, the different distinct categories of clinical samples of the same nature for the same reference microbe preferably corresponding to clinical samples whose components other than the microbe within said clinical sample are qualitatively and/or quantitatively different inducing spectral differences of all or part of the characteristic peaks of the proteins or protein fragments from said reference microbe.

4. The process as claimed in claim 3 **characterized in that** said reference spectra are obtained from different distinct categories of urine samples comprising different concentrations of white blood cells and/or red blood cells, preferably at least:
- a first group of urine samples containing not more than 10 white blood cells and/or red blood cells/µl, and
- a second group of urine samples containing more than 10 white blood cells and/or red blood cells/µl.

5. The process as claimed in one of claims 1 to 4 **characterized in that** said reference microbes are selected from bacteria and yeasts, preferably bacteria.

6. The process as claimed in claim 5 **characterized in that**, for urine samples, said reference bacteria comprise at least *Escherichia coli, Klebsiella pneumoniae, Enterococcus faecalis, Proteus mirabilis, Pseudomonas aeruginosa.*

7. The process as claimed in claim 5 or 6 **characterized in that** said reference bacteria are selected from at least *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii.*

8. The process as claimed in one of claims 1 to 7 **characterized in that** said reference microbes are selected from at least: *Escherichia coli, Klebsiella pneumoniae, Enterococcus faecalis, Pseudomonas aeruginosa, Proteus mirabilis, Citrobacter koseri, Enterobacter aerogenes, Enterobacter cloacae, Enterococcus faecium, Klebsiella oxytoca, Morganella morganii, Staphylococcus aureus, Staphylococcus saprophyticus, Streptococcus agalactiae, Serratia marcescens, Citrobacter freundii, Staphylococcus epidermidis, Stenotrophomonas maltophilia, Raoultella ornithinolytica, Gardnerella vaginalis, Hafnia alvei, Acinetobacter pittii, Pseudomonas putida Serratia marcescens*, *Klebsiella oxytoca, Aerococcus urinae, Staphylococcus capitis ; Pseudomonas putida, Acinetobacter nosocomialis, Enterobacter kobei, Streptococcus oralis, Enterococcus gallinarum, Stenotrophomonas maltophilia, Raoultella ornithinolytica, Pseudomonas mosselii, Hafnia alvei, Providencia stuartii, Corynebacterium amycolatum, Corynebacterium striatum, Streptococcus anginosus, Acinetobacter baumannii, Staphylococcus hominis ssp hominis, Gardnerella vaginalis, Candida albicans and Candida glabrata.*

9. The process as claimed in one of claims 1 to 8 **characterized in that** said reference library further comprises at least one protein extract reference spectrum of said healthy clinical specimen not infected by a microbe.

10. The process as claimed in one of claims 1 to 9, **characterized in that** said reference bank comprises at least 500 reference spectra made from at least 500 samples of said clinical specimens each containing one said reference microbe, preferably all of said clinical reference specimens containing at least 10 different said reference microbes, preferably at least 20 different said reference microbes.

11. The process as claimed in one of claims 1 to 10 **characterized in that** said protein extracts are obtained by carrying out a preparation process comprising the following successive steps:
1) the liquid is removed from said clinical specimen to be analyzed, and a first solid residue is recovered, and
2) preferably a detergent solution is added to said first solid residue, and
3) the liquid is removed again and a second solid residue is recovered, and
4) a lysis of the cells present in said second solid residue is carried out, preferably mechanical lysis, and
5) the proteins and protein fragments present in said second solid residue are solubilized with a solvent solution, and
6) the non-solubilized substances are removed.

12. The process as claimed in claim 11 **characterized in that** said protein extracts are obtained by carrying out a preparation process comprising the following successive steps:
1) the liquid is removed from said clinical specimen to be analyzed by performing a first centrifugation and removing the centrifugation supernatant, and recovering a first solid residue which is the first centrifugation pellet, and
2) a detergent solution is added to said first solid residue, preferably a saponin solution, and
3) a second solid residue is recovered by a second centrifugation and by removing the supernatant and recovering a second solid residue which is the second centrifugation pellet, and
4) mechanical lysis of said second solid residue is carried out by mechanical grinding to obtain fragments of a size less than or equal to 1 µm, preferably using glass microbeads, and
5) the proteins and protein fragments present in said second solid residue of cell lysate are solubilized, preferably with a solution of trifluoroacetic acid and acetonitrile, and
6) the non-solubilized substances are removed by a third centrifugation and by removing the pellet and recovering the supernatant from the third centrifugation.

13. The process as claimed in one of claims 1 to 12 **characterized in that** volumes of clinical urine samples of less than 3 mL preferably not more than 1 mL are used and the bacterial concentrations of said urine samples of the reference spectra and said urine sample to be analyzed are 10⁴ to 10⁷ bacteria per milliliter.
